(19) Europäisches Patentamt / European Patent Office / Office européen des brevets

(11) **EP 4 729 611 A1**

(12) **EUROPEAN PATENT APPLICATION**
published in accordance with Art. 153(4) EPC

(43) Date of publication:
**22.04.2026 Bulletin 2026/17**

(21) Application number: **24823489.0**

(22) Date of filing: **14.06.2024**

(51) International Patent Classification (IPC):
*C12N 9/80* (2006.01)          *C12N 9/52* (2006.01)
*C12N 9/54* (2006.01)

(52) Cooperative Patent Classification (CPC):
**C12N 9/52; C12N 9/54; C12N 9/80**

(86) International application number:
**PCT/JP2024/021776**

(87) International publication number:
**WO 2024/257876 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.06.2023  JP 2023098146**

(71) Applicant: **Amano Enzyme Inc.
Nagoya-shi
Aichi 460-8630 (JP)**

(72) Inventor: **YACHI, Hiroyuki
Kakamigahara-shi, Gifu 509-0109 (JP)**

(74) Representative: **Vossius & Partner
Patentanwälte Rechtsanwälte mbB
Siebertstraße 3
81675 München (DE)**

(54) **LIQUID ENZYME PREPARATION**

(57)   The present invention provides a liquid enzyme preparation containing a protein deamidase and having excellent activity stability, the liquid enzyme preparation comprising the protein deamidase together with another enzyme. A liquid enzyme preparation comprising a protein deamidase and a protease is excellent with respect to the stability of the activity of the protein deamidase.

**Description**

Technical Field

The present invention relates to a liquid enzyme preparation. More specifically, the present invention relates to a liquid enzyme preparation containing a protein deamidase and having excellent activity stability of the protein deamidase.

Background Art

As an enzyme that deamidates a γ-amide group and a β-amide group of a glutamine residue and an asparagine residue in a protein, as described in PTL 1 and the like, a protein glutaminase derived from Chryseobacterium gleum JCM2410 strain and a protein glutaminase derived from Chryseobacterium proteolyticum 9670 strain are known. Enzyme preparation products of such protein deamidases are currently only commercially available in a powder form.

As for the enzyme preparation, generally, a liquid form is more desirable than a powder form in consideration of dusting and handling. For this reason, a technique for formulating an enzyme preparation in a liquid form has been studied for various enzymes. For example, PTL 2 proposes a liquid enzyme preparation containing at least one milk protein crosslinking and/or modifying enzyme in polyol-water suspension containing 25% to 100% (w/w) polyol and having a pH value within a range from 4.4 to 5.1. Specifically, PTL 2 discloses that the stability of transglutaminase at a pH of 5.2 is low, and based on this finding, the stability of a transglutaminase preparation in a glycerol-water suspension with a pH of 4.4 to 4.8 and a transglutaminase preparation in a sorbitol-water suspension with a pH of 4.6 are improved, and in addition thereto, also discloses a tyrosinase preparation in a glycerol-water suspension with a pH of 4.6 and a protein glutaminase preparation in a glycerol-water suspension with a pH of 4.6.

PTL 3 describes that in a useful protein composition containing a useful protein, a solvent, and a compound having a thiol structure, a useful protein composition such as a stable interferon can be produced by bubbling an asphyxiant gas such as nitrogen to set the concentration of dissolved oxygen to 3 mg/L or less.

Citation List

Patent Literatures

PTL 1: WO 2010/029685 A
PTL 2: WO 2013/064736A
PTL 3: Japanese Patent Laid-open Publication No. 2013-049650

Summary of Invention

Technical Problem

In PTL 2, the stability of the liquid enzyme preparation of the transglutaminase preparation has been studied, while a liquid enzyme preparation of a protein deamidase has been only disclosed as a preparation formulation, and the stability of this enzyme has not been sufficiently studied. In PTL 3, while the antiviral activity of an aqueous preparation containing canine interferon-γ is examined, there is no mention of a liquid enzyme preparation of a protein deamidase, and naturally no examination is made. Some enzyme preparations are composed by combining a plurality of enzymes, but the stability of a protein deamidase has not been studied for a liquid enzyme preparation containing the protein deamidase together with other enzymes.

Therefore, an object of the present invention is to provide a liquid enzyme preparation containing a protein deamidase together with other enzymes, the liquid enzyme preparation having excellent activity stability of the protein deamidase. Solution to Problem

As a result of intensive studies, the present inventor has found that a liquid enzyme preparation containing a protein deamidase is excellent in activity stability of the protein deamidase even when the liquid enzyme preparation contains a protease.

The present inventor has further found that in a liquid enzyme preparation containing a protein deamidase and a protease, the activity stability of the protein deamidase is further improved when the liquid enzyme preparation is accommodated in a container whose headspace is replaced with an inert gas, when the upper limit of the protease content is limited to a predetermined amount, and/or when sorbitol is blended in the presence of a predetermined amount or more of protease, and even when a citrate is blended in the presence of a predetermined amount or more of protease, excellent activity stability of the protein deamidase is obtained.

The present invention has been completed based on these findings. That is, the present invention includes the following inventions.

Item 1. A liquid enzyme preparation containing: (A) a protein deamidase; and (B) a protease.

Item 2. The liquid enzyme preparation described in item 1, in which the liquid enzyme preparation is accommodated in a container whose headspace is replaced with an inert gas.

Item 3. The liquid enzyme preparation described in item 1 or 2, further containing (C) sorbitol.

Item 4. The liquid enzyme preparation described in any one of items 1 to 3, further containing (D) a citrate.

Item 5. The liquid enzyme preparation described in item 3, in which a content of the component (C) is 30 w/v% or more.

Item 6. The liquid enzyme preparation described in item 3 or 4, in which a content of the component (D) is 1 w/v% or more.

Item 7. The liquid enzyme preparation described in any one of items 1 to 6, in which a pH is 5.5 or more.

Item 8. The liquid enzyme preparation described in any one of items 1 to 7, in which a pH is 8.2 or less.

Item 9. The liquid enzyme preparation described in any one of items 1 to 8, in which a content of the component (A) is 0.1 to 10,000 U/ml.

Item 10. The liquid enzyme preparation described in any one of items 1 to 9, in which the component (A) is a protein deamidase derived from Chryseobacterium proteolyticum.

Item 11. The liquid enzyme preparation described in any one of items 1 to 10, in which a content of the component (B) is 0.01 to 2,000 U/mL.

Item 12. The liquid enzyme preparation described in any one of items 1 to 11, in which the component (B) is a bacteria-derived protease.

Item 13. The liquid enzyme preparation described in item 12, in which the bacteria-derived protease is a protease derived from the genus Chryseobacterium.

Item 14. The liquid enzyme preparation described in item 12, in which the bacteria-derived protease is a protease derived from the genus Bacillus and/or Geobacillus.

Item 15. The liquid enzyme preparation described in any one of items 1 to 14, in which a content of the component (B) per 100 U of the component (A) is 0.002 to 400 U.

Advantageous Effects of Invention

According to the present invention, there is provided a liquid enzyme preparation having excellent activity stability of a protein deamidase.

Brief Description of Drawing

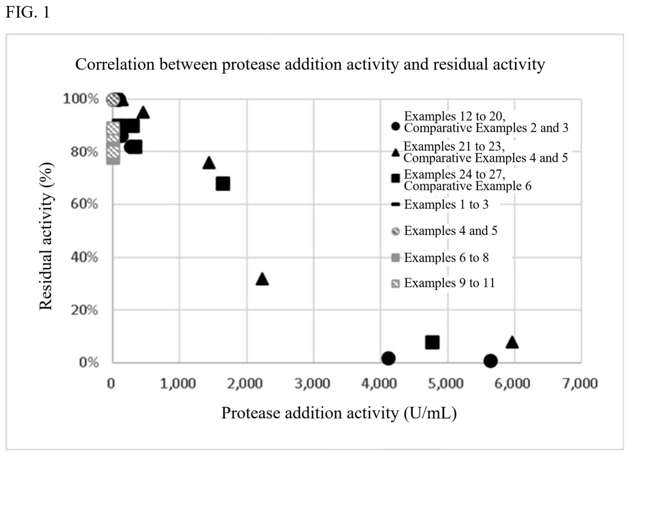

[Fig. 1] Fig. 1 shows a relationship between an amount of a protease added to a liquid enzyme preparation containing a protein deamidase (addition activity) and residual activity of the protein deamidase of the liquid enzyme preparation. Description of Embodiments

[1. Liquid Enzyme Preparation]

A liquid enzyme preparation of the present invention contains (A) a protein deamidase (hereinafter, also referred to as "component (A)") and (B) a protease (hereinafter, also referred to as "component (B)"). Hereinafter, the liquid enzyme preparation of the present invention will be described in detail. The liquid enzyme preparation of the present invention can further contain (C) sorbitol (hereinafter, also referred to as "component (C)") and/or (D) a citrate (hereinafter, also referred to as "component (D)").

[1-1. (A) Protein deamidase]

The liquid enzyme preparation of the present invention contains a protein deamidase as the component (A). The type, origin, and the like of the protein deamidase are not particularly limited as long as the protein deamidase is an enzyme that exhibits an action of decomposing an amide group-containing side chain of a protein without cleaving peptide bonds and without crosslinking the protein. As long as the above action is main activity, the protein deamidase may further have an action of decomposing an amide group-containing side chain of a protein with cleaving peptide bonds and crosslinking the protein. Examples of the protein deamidase include an enzyme that deamidates a glutamine residue in a protein and converts it into glutamic acid (for example, protein glutaminase), and an enzyme that deamidates an asparagine residue in a protein and converts it into aspartic acid (for example, protein asparaginase). More specific examples of the protein deamidase include commercially available products of a protein deamidase derived from the genus Chryseobacterium, Flavobacterium, Empedobacter, Sphingobacterium, Aureobacterium, or Myroides, which is disclosed in JP 2000-50887 A, JP 2001-218590 A, and WO 2006/075772 A1; a protein deamidase derived from the genus Luteimicrobium, Agromyces, Microbacterium, or Leifsonia, which is disclosed in WO 2015/133590 A1; and a protein glutaminase derived from the genus Chryseobacterium. These protein deamidases may be used singly or in combination of a plurality of kinds thereof.

Among these protein deamidases, from the viewpoint of obtaining a liquid enzyme preparation having further excellent activity stability, a protein deamidase derived from the genus Chryseobacterium is preferable, a protein glutaminase derived from the genus Chryseobacterium is more preferable, and a protein glutaminase derived from Chryseobacterium proteolyticum species is further preferable.

The protein deamidase can be prepared from a culture broth of a microorganism from which the protein deamidase is derived. Specific examples of the preparation method include a method of recovering a protein deamidase from a culture broth or a bacterial cell of the above-mentioned microorganism. For example, in the case of using a microorganism that secretes a protein deamidase , an enzyme can be separated and/or purified after recovering bacterial cells from the culture broth in advance by filtration, a centrifugal treatment, or the like, as necessary. In the case of using a microorganism that does not secrete a protein deamidase, an enzyme can be separated and/or purified after recovering bacterial cells from the culture broth in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include a centrifugal separation method, a UF concentration method, a salting-out method, and various chromatography methods or the like using an ion-exchange resin or the like.

The content of the component (A) in the liquid enzyme preparation of the present invention is not particularly limited, and is, for example, 0.1 to 10,000 U/mL, 1 to 5,000 U/mL, 10 to 3,000 U/mL, or 50 to 2,000 U/mL, preferably 100 to 1,500 U/mL, more preferably 200 to 1,000 U/mL, further preferably 300 to 900 U/mL or 350 to 700 U/mL, and even more preferably 400 to 600 U/mL.

For the activity of the protein deamidase, benzyloxycarbonyl-L-glutaminylglycine (Z-Gln-Gly) is used as a substrate, and the amount of enzyme that liberates 1 $\mu$mol of ammonia per minute is defined as 1 unit (1 U).

[1-2. (B) Protease]

The liquid enzyme preparation of the present invention contains a protease as the component (B). In the present invention, the origin of the protease is not particularly limited, and examples thereof include a bacteria-derived protease.

Examples of the bacteria-derived protease include proteases derived from the genera Chryseobacterium, Bacillus, Geobacillus, and the like.

The protease derived from the genus Chryseobacterium is not particularly limited as long as it can obtain the desired effect of the present invention. Specific examples of the protease derived from the genus Chryseobacterium include proteases derived from Chryseobacterium nematophagum, Chryseobacterium cucumeris, Chryseobacterium lactis, Chryseobacterium rhizoplanae, Chryseobacterium joostei, Chryseobacterium shigense, Chryseobacterium proteolyticum, Chryseobacterium gleum, Chryseobacterium soli, and the like. These proteases derived from the genus Chryseobacterium may be used singly or in combination of a plurality of kinds thereof. Among these proteases derived from the genus Chryseobacterium, proteases derived from Chryseobacterium proteolyticum species are preferable from the viewpoint of further enhancing the activity stabilizing effect of the protein deamidase.

The protease derived from the genus Chryseobacterium can be prepared by a known method. As an example, the protease can be easily prepared by a method of culturing bacteria belonging to the genus Chryseobacterium and separating the protease using a known means, a method using a genetic recombination technique, or the like.

Specific examples of the protease derived from the genus Bacillus (or Geobacillus) include proteases derived from Bacillus amyloliquefaciens, Bacillus cereus, Bacillus clausii, Bacillus intermedius, Bacillus lentus, Bacillus licheniformis, Bacillus stearothermophilus, Bacillus subtilis, Bacillus thermoproteolyticus, and corresponding genus Geobacillus. In the present invention, one kind of the protease may be used singly, or a plurality of kinds thereof may be used in combination. Among these proteases derived from the genus Bacillus (or Geobacillus), from the viewpoint of further enhancing the activity stabilizing effect of the protein deamidase, a protease derived from Bacillus licheniformis, a protease derived from Bacillus amyloliquefaciens, and a protease derived from corresponding genus Geobacillus are preferable.

The content of the component (B) in the liquid enzyme preparation of the present invention is not particularly limited as long as the effects of the present invention are not impaired, and is, for example, 0.01 to 10,000 U/mL or 0.01 to 5,000 U/mL.

From the viewpoint of further enhancing the activity stabilizing effect of the protein deamidase, the content of the component (B) in the liquid enzyme preparation of the present invention is preferably 0.01 to 3,500 U/mL, more preferably 0.01 to 3,000 U/mL or 0.01 to 2,500 U/mL, further preferably 0.01 to 2,000 U/mL, 0.01 to 1,800 U/mL, 0.01 to 1,650 U/mL, 0.01 to 1,500 U/mL, 0.01 to 1,400 U/mL, 0.01 to 1,300 U/mL, 0.01 to 1,200 U/mL, 0.01 to 1,100 U/mL, 0.01 to 1,000 U/mL, 0.01 to 900 U/mL, 0.01 to 800 U/mL, 0.01 to 700 U/mL, 0.01 to 600 U/mL, 0.01 to 500 U/mL, 0.01 to 400 U/mL, 0.01 to 300 U/mL, 0.01 to 200 U/mL, or 0.01 to 150 U/mL, and even more preferably 0.01 to 100 U/mL, 0.01 to 80 U/mL, 0.01 to 75 U/mL, 0.01 to 70 U/mL, 0.01 to 65 U/mL, 0.01 to 40 U/mL, 0.01 to 20 U/mL, 0.01 to 10 U/mL, or 0.01 to 5 U/mL (when the protease is a protease derived from the genus Bacillus (or Geobacillus), even more preferably 0.01 to 20 U/mL, 0.01 to 10 U/mL, or 0.01 to 5 U/mL).

From the viewpoint of effectively obtaining the activity stabilizing effect of the protein deamidase, the content of the component (B) in the liquid enzyme preparation of the present invention may be 0.1 to 2,000 U/mL, 0.5 to 2,000 U/mL, 1 to 2,000 U/mL, 5 to 2,000 U/mL, 10 to 2,000 U/mL, 15 to 2,000 U/mL, 20 to 2,000 U/mL, 30 to 2,000 U/mL, 40 to 2,000 U/mL, 50 to 2,000 U/mL, 60 to 2,000 U/mL, 70 to 2,000 U/mL, 80 to 2,000 U/mL, 90 to 2,000 U/mL, 100 to 2,000 U/mL, 150 to 2,000 U/mL, 200 to 2,000 U/mL, 250 to 2,000 U/mL, or 300 to 2,000 U/mL.

From the viewpoint of enhancing the activity stabilizing effect of the protein deamidase by blending the component (C), the content of the component (B) in the liquid enzyme preparation of the present invention is preferably 100 to 2,000 U/mL, 150 to 2,000 U/mL, 200 to 2,000 U/mL, 250 to 2,000 U/mL, or 300 to 2,000 U/mL.

The content of the component (B) per 100 U of the component (A) in the liquid enzyme preparation of the present invention is not particularly limited as long as the effects of the present invention are not impaired, and is, for example, 0.002 to 2,000 U or 0.002 to 1,000 U.

From the viewpoint of further enhancing the activity stabilizing effect of the protein deamidase, the content of the component (B) per 100 U of the component (A) in the liquid enzyme preparation of the present invention is preferably 0.002 to 700 U, more preferably 0.002 to 600 U or 0.002 to 500 U, further preferably 0.002 to 400 U, 0.002 to 360 U, 0.002 to 330 U, 0.002 to 300 U, 0.002 to 280 U, 0.002 to 260 U, 0.002 to 240 U, 0.002 to 220 U, 0.002 to 200 U, 0.002 to 180 U, 0.002 to 160 U, 0.002 to 140 U, 0.002 to 120 U, 0.002 to 100 U, 0.002 to 80 U, 0.002 to 60 U, 0.002 to 40 U, or 0.002 to 30 U, and even more preferably 0.002 to 20 U, 0.002 to 16 U, 0.002 to 15 U, 0.002 to 14 U, 0.002 to 13 U, 0.002 to 8 U, 0.002 to 4 U, 0.002 to 2 U, or 0.002 to 1 U (when the protease is a protease derived from the genus Bacillus (or Geobacillus), even more preferably 0.002 to 4 U, 0.002 to 2 U, or 0.002 to 1 U).

From the viewpoint of effectively obtaining the activity stabilizing effect of the protein deamidase, the content of the component (B) per 100 U of the component (A) in the liquid enzyme preparation of the present invention may be 0.02 to 400 U, 0.1 to 400 U, 0.2 to 400 U, 1 to 400 U, 2 to 400 U, 3 to 400 U, 4 to 400 U, 6 to 400 U, 8 to 400 U, 10 to 400 U, 12 to 400 U, 14 to 400 U, 16 to 400 U, 18 to 400 U, 20 to 400 U, 30 to 400 U, 40 to 400 U, 50 to 400 U, or 60 to 400 U.

From the viewpoint of enhancing the activity stabilizing effect of the protein deamidase by blending the component (C), the content of the component (B) per 100 U of the component (A) in the liquid enzyme preparation of the present invention is preferably 20 to 400 U, 30 to 400 U, 40 to 400 U, 50 to 400 U, or 60 to 400 U.

The protease activity is measured using casein as a substrate by the Folin method. That is, the protease activity is determined in such a manner that an enzymatic reaction is performed using casein as a substrate at a pH set according to the optimum pH of a protease to be measured by a conventional method, and an amount of an enzyme which causes an increase in colored materials by Folin's reagent corresponding to 1 $\mu$g of tyrosine per minute is defined as 1 unit (1 U).

[1-3. (C) Sorbitol]

The liquid enzyme preparation of the present invention can contain sorbitol as the component (C). In the liquid enzyme preparation of the present invention, a case where the component (C) may be blended is not particularly limited.

The component (C) is preferable in that the stability of the liquid enzyme preparation can be further improved particularly when the content of the component (B) in the liquid enzyme preparation of the present invention is 100 to 2,000 U/mL, 150 to 2,000 U/mL, 200 to 2,000 U/mL, 250 to 2,000 U/mL, or 300 to 2,000 U/mL, or when the content of the component (B) per 100 U of the component (A) is 20 to 400 U, 30 to 400 U, 40 to 400 U, 50 to 400 U, or 60 to 400 U.

The content of the component (C) in the liquid enzyme preparation of the present invention is, for example, 10 w/v% or more or 15 w/v% or more, preferably 20 w/v% or more or 25 w/v% or more, more preferably 30 w/v% or more, further preferably 35 w/v% or more, and even more preferably 38 w/v% or more. In this case, the upper limit of the content range of the component (C) is also not particularly limited, and is, for example, 80 w/v% or less, 75 w/v% or less, 65 w/v% or less, 55 w/v% or less, 50 w/v% or less, 45 w/v% or less, or 42 w/v% or less. The specific range of the content of the component (C) is, for example, 10 to 80 w/v% or 15 to 75 w/v%, preferably 20 to 65 w/v% or 25 to 55 w/v%, more preferably 30 to 50 w/v%, further preferably 35 to 45 w/v%, and even more preferably 38 to 42 w/v%.

The content of the component (C) per 1 U of the component (A) in the liquid enzyme preparation of the present invention is, for example, 0.15 mg or more, 0.2 mg or more, 0.3 mg or more, 0.4 mg or more, or 0.45 mg or more, preferably 0.5 mg or more or 0.6 mg or more, and more preferably 0.75 mg or more. The upper limit of the content range of the component (C) per 1 U of the component (A) in the liquid enzyme preparation of the present invention is also not particularly limited, and is, for example, 1.2 mg or less, 1.1 mg or less, 1 mg or less, 0.85 mg or less, or 0.83 mg or less. The specific range of the content of the component (C) per 1 U of the component (A) is, for example, 0.15 to 1.2 mg, 0.2 to 1.1 mg, 0.3 to 1 mg, 0.4 to 1 mg, or 0.45 to 1 mg, preferably 0.5 to 0.85 mg or 0.6 to 0.85 mg, and more preferably 0.75 to 0.83 mg.

[1-4. (D) Citrate]

The liquid enzyme preparation of the present invention can contain a citrate as the component (D). Specific examples of the citrate preferably include alkali metal salts such as a potassium salt and a sodium salt, and more preferably include a sodium salt.

In the liquid enzyme preparation of the present invention, a case where the component (D) may be blended is not particularly limited. The component (D) can be used as a pH buffer. The component (D) tends to reduce the stability of the liquid enzyme preparation of the present invention while suppressing pH variation. However, the component (D) is preferable in that the decrease in stability of the liquid enzyme preparation can be suppressed particularly when the content of the component (B) in the liquid enzyme preparation of the present invention is 80 to 2,000 U/mL, 90 to 2,000 U/mL, 100 to 2,000 U/mL, 150 to 2,000 U/mL, 200 to 2,000 U/mL, 250 to 2,000 U/mL, or 300 to 2,000 U/mL, or when the content of the component (B) per 100 U of the component (A) is 16 to 400 U, 18 to 400 U, 20 to 400 U, 30 to 400 U, 40 to 400 U, 50 to 400 U, or 60 to 400 U.

The content of the component (D) in the liquid enzyme preparation of the present invention is, for example, 0.5 w/v% or more, preferably 0.8 w/v% or more, more preferably 1 w/v% or more, and further preferably 1.5 w/v% or more, 2 w/v% or more, 2.5 w/v% or more, or 2.8 w/v% or more. In this case, the upper limit of the content range of the component (D) is also not particularly limited, and is, for example, 20 w/v% or less, 18 w/v% or less, 16 w/v% or less, 13 w/v% or less, 11 w/v% or less, 9 w/v% or less, 7 w/v% or less, 5 w/v% or less, 4 w/v% or less, or 3.5 w/v% or less. The specific range of the content of the component (D) is, for example, 0.5 to 20 w/v% or 0.5 to 18 w/v%, preferably 0.8 to 16 w/v% or 0.8 to 13 w/v%, more preferably 1 to 11 w/v% or 1 to 9 w/v%, and further preferably 1.5 to 7 w/v%, 2 to 5 w/v%, 2.5 to 4 w/v%, or 2.8 to 3.5 w/v%.

The content of the component (D) per 1 U of the component (A) in the liquid enzyme preparation of the present invention is, for example, 0.0075 mg or more, preferably 0.012 mg or more, more preferably 0.02 mg or more, and further preferably 0.025 mg or more, 0.03 mg or more, 0.035 mg or more, 0.04 mg or more, 0.045 mg or more, or 0.05 mg or more. The upper limit of the content range of the component (D) per 1 U of the component (A) in the liquid enzyme preparation of the present invention is also not particularly limited, and is, for example, 0.3 mg or less, 0.27 mg or less, 0.24 mg or less, 0.2 mg or less, 0.16 mg or less, 0.14 mg or less, 0.1 mg or less, 0.09 mg or less, 0.08 mg or less, or 0.07 mg or less. The specific range of the content of the component (D) per 1 U of the component (A) is 0.0075 to 0.3 mg, preferably 0.012 to 0.27 mg, more preferably 0.02 to 0.24 mg, and further preferably 0.025 to 0.2 mg, 0.03 to 0.16 mg, 0.035 to 0.14 mg, 0.04 to 0.1 mg, 0.045 to 0.09 mg, 0.045 to 0.08 mg, or 0.05 to 0.07 mg.

[1-5. Other Components]

The liquid enzyme preparation of the present invention contains water as a base in addition to the component (A) and the component (B) described above, and the component (C) and/or the component (D) blended as necessary. The liquid enzyme preparation of the present invention may or may not contain other components, in addition to the component (A), the component (B), and water, and the component (C) and/or the component (D) blended as necessary, to the extent that the effects of the present invention are not affected. Examples of the other component include other enzymes that are other than the component (A) and the component (B), and other additives that are other than the component (C) and the component (D) and the like.

Examples of the other enzymes include amylase (α-amylase, β-amylase, or glucoamylase), glucosidase (α-glucosidase or β-glucosidase), galactosidase (α-galactosidase or β-galactosidase), peptidase (leucine peptidase or aminopeptidase), lipase, esterase, cellulase, phosphatase (acid phosphatase or alkaline phosphatase), nuclease, deaminase, oxidase, dehydrogenase, glutaminase, pectinase, catalase, dextranase, transglutaminase, pullulanase, and the like. These other enzymes may be contained singly or in combination of a plurality of kinds thereof.

Examples of the other additives include a buffer, a suspending agent, physiological saline, and the like. Examples of the buffer include a phosphate buffer and an acetate buffer. These other additives may be contained singly or in combination of a plurality of kinds thereof.

[1-6. Preparation Form etc.]

The liquid enzyme preparation of the present invention is a liquid enzyme composition. The liquid enzyme preparation of the present invention can be prepared by adding a protein deamidase, a protease, a predetermined additive, and other components blended as necessary in predetermined amounts to water, and further sterilizing the mixture as necessary.

The pH (referring to pH at 25°C; the same applies hereinafter) of the liquid enzyme preparation of the present invention is not particularly limited, and is, for example, 5.5 or more and preferably 5.8 or more. The upper limit of the pH range of the liquid enzyme preparation of the present invention is not particularly limited, and is preferably 8.2 or less, more preferably 7.5 or less, further preferably 7 or less, and even more preferably 6.5 or less, and the specific range of the pH is, for example, 5.5 to 8.2, preferably 5.5 to 7.5, more preferably 5.8 to 7, and even more preferably 5.8 to 6.5.

[1-7. Dissolved Oxygen Amount]

The dissolved oxygen amount contained in the liquid enzyme preparation of the present invention is not particularly limited. In particular, since the liquid enzyme preparation of the present invention is excellent in the activity stability of the protein deamidase, an activity stabilizing effect can be effectively obtained even when dissolved oxygen amount contained in the liquid enzyme preparation is not actively reduced. From such a viewpoint, a suitable example of the dissolved oxygen amount in the liquid enzyme preparation is more than 3 mg/L, preferably more than 4 mg/L, further preferably 4.5 mg/L or more, and even more preferably 5 mg/L or more. The upper limit of the range of the dissolved oxygen amount in the liquid enzyme preparation is not particularly limited, and is, for example, 8 mg/L or less, preferably 7 mg/L or less, more preferably 6 mg/L or less, and even more preferably 5.5 mg/L or less. The specific range of the dissolved oxygen amount is more than 3 mg/L and 8 mg/L or less, preferably more than 4 mg/L and 7 mg/L or less, further preferably 4.5 to 6 mg/L, and even more preferably 5 to 5.5 mg/L. Note that the liquid enzyme preparation of the present invention does not exclude a case where the dissolved oxygen amount is 3 mg/L or less.

[1-8. Accommodating in Container]

The liquid enzyme preparation of the present invention is appropriately accommodated in a container capable of accommodating a liquid.

Preferably, the liquid enzyme preparation of the present invention is accommodated in a container whose headspace is replaced with an inert gas. The inert gas is not particularly limited, and examples thereof include a nitrogen gas and a rare gas (such as helium gas or argon gas). A method of replacing the headspace of the container with an inert gas is not particularly limited, and examples thereof include a method of directly adding a gaseous inert gas or a liquid inert gas into the container.

The liquid enzyme preparation of the present invention can improve the activity stability of the protein deamidase by being accommodated in a container whose headspace is replaced with an inert gas without bubbling an inert gas into the liquid enzyme preparation. Therefore, in a preferred embodiment of the liquid enzyme preparation of the present invention, an inert gas is not bubbled. Note that when the liquid enzyme preparation of the present invention is accommodated in a container whose headspace is replaced with an inert gas, the form of bubbling the inert gas is not excluded.

In the liquid enzyme preparation of the present invention accommodated in the container, the oxygen concentration in the container (that is, in the gas phase in the container) is, for example, 5 v/v% or less, preferably 3 v/v% or less, and more preferably 2 v/v% or less, 1.5 v/v% or less, 1.3 v/v% or less, or 1 v/v% or less. The oxygen concentration range in the container is not particularly limited at the lower limit, and is, for example, 0.01 v/v% or more, 0.05 v/v% or more, 0.1 v/v% or more, 0.4 v/v% or more, 0.6 v/v% or more, 0.8 v/v% or more, or 1 v/v% or more.

[1-9. Use Application]

As the use application of the liquid enzyme preparation of the present invention, the liquid enzyme preparation can be used for any use application utilizing at least protein deamidase activity. For example, the liquid enzyme preparation of the present invention can be used for the purpose of modifying a protein. A specific embodiment of the modification of the protein is not particularly limited, and any modification may be used as long as it utilizes a change in properties of the protein caused by generation of a carboxyl group by deamidation of a $\gamma$-amide group and a $\beta$-amide group of a glutamine residue and an asparagine residue of the protein. Specifically, examples of the modification of the protein include an increase in solubility of the protein, an increase in water dispersibility, an improvement in emulsifying power, an improvement in emulsion stability, and the like.

Therefore, the liquid enzyme preparation of the present invention can be widely used in the food field. Specific examples of the use application include such as use application for improving solubility, dispersibility, emulsifiability, and the like of an animal protein and/or a plant protein in a weakly acidic condition environment in a pH range of normal foods (for example, use application for producing of acidic beverages such as coffee/whiteners and juices, dressings, mayonnaise, and creams); increase in solubility and dispersibility of a poorly soluble plant protein (for example, use application for producing a fried food powder or the like using wheat gluten); use application for modifying dough in breadmaking and baking (for example, use application for producing crackers, biscuits, cookies, pizza, or pie crust); use application for removing or reducing allergen in allergenic protein in food (for example, use application for producing food for wheat allergic patients); use application for reducing mineral sensitivity of a protein, increasing a soluble mineral content in a liquid containing a protein and mineral, and enhancing absorbability of the mineral into a human body (for example, use application for producing highly mineral (for example, calcium)-containing beverages, mineral (for example, calcium) absorption enhancers); use application for reducing bitterness, and/or use application for enhancing the glutamic acid content (for example, amino acid-based seasoning (hydrolyzate of animal protein (HAP), hydrolyzate of plant protein (HVP)), and use application for producing fermented soybean paste or soy sauce).

As the use application of the liquid enzyme preparation of the present invention, the liquid enzyme preparation can be used for any use application utilizing both protein deamidase activity and protease activity.

Examples

Hereinafter, the present invention will be more specifically described by means of Examples; however, the present invention is not limited to these Examples.

[(A) Protein Deamidase]

A culture broth of Chryseobacterium proteolyticum species, which was a protein deamidase (protein glutaminase) producing strain, was subjected to removal of insoluble components such as extraneous macromolecules, and purified by ion exchange chromatography. The protein deamidase activity was measured by the following method.

To 1 mL of a 0.2 M phosphate buffer (pH 6.5) containing 30 mM Z-Gln-Gly, 0.1 mL of a sample solution containing a protein deamidase was added, the mixture was incubated at 37°C for 10 minutes, and then 1 mL of a 0.4 M TCA solution was added to stop the reaction. As a blank, to 1 mL of a 0.2 M phosphate buffer (pH 6.5) containing 30 mM Z-Gln-Gly, 1 mL of a 0.4 M TCA solution was added, 0.1 mL of a sample solution containing a protein deamidase was further added, and the mixture was left to stand at 37°C for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The activity of the protein deamidase was calculated from the following formula with the amount of enzyme that produces 1 $\mu$mol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

[Mathematical Formula 1]

$$\text{Protein deamidase activity (U/mL)}$$

$$= \text{Ammonia concentration in reaction solution (mg/L)} \times (1/17.03) \times (\text{Reaction solution amount/Enzyme solution amount}) \times (1/10) \times \text{Df}$$

[(B) Protease]

Proteases used in the following Test Examples are as follows.

- Protease derived from the genus Chryseobacterium: Protease derived from Chryseobacterium proteolyticum
- Protease derived from Bacillus licheniformis
- Protease derived from Bacillus amyloliquefaciens

The protease activity was measured by the following method.

After 5 mL of a 0.6% (w/v) casein solution (0.05 mol/L of sodium hydrogen phosphate, pH 8.0) was warmed at 37°C for 10 minutes, 1 mL of a sample solution containing a protease was added, and the mixture was immediately shaken. After this solution was incubated at 37°C for 10 minutes, 5 mL of a trichloroacetic acid reagent (containing 1.8 (w/v)% trichloroacetic acid, 1.8 (w/v)% sodium acetate, and 0.33 mol/L acetic acid) was added, the mixture was shaken, and left to stand at 37°C for 30 minutes again, and the mixture was filtered. The first filtrate (3 mL) was removed, the next filtrate (2 mL) was weighed, 5 mL of a 0.55 mol/L sodium carbonate reagent and 1 mL of Folin's reagent (1 → 3) were added, and the mixture was shaken well and left to stand at 37°C for 30 minutes. An absorbance AT of this solution (enzymatic reaction solution) at a wavelength of 660 nm was measured using water as a control.

Separately, a solution (blank) was prepared by performing the same operation as in the enzymatic reaction solution except that 1 mL of a sample solution containing a protease was weighed, 5 mL of a trichloroacetic acid reagent (containing 1.8 (w/v)% trichloroacetic acid, 1.8 (w/v)% sodium acetate, and 0.33 mol/L acetic acid) was added, the mixture was shaken, 5 mL of a 0.6% (w/v) casein solution (0.05 mol/L sodium hydrogen phosphate, pH 8.0) was then added, the mixture was immediately shaken and incubated at 37°C for 30 minutes, and an absorbance AB of this solution was measured.

Each of 1 mL, 2 mL, 3 mL, and 4 mL of a 1 mg/mL tyrosine standard stock solution (0.2 mol/L of hydrochloric acid) was weighed, and a 0.2 mol/L hydrochloric acid reagent was added thereto to make 100 mL. Each solution (2 mL) was weighed, 5 mL of a 0.55 mol/L sodium carbonate reagent and 1 mL of Folin's reagent (1 → 3) were added, and the mixture was immediately shaken and incubated at 37°C for 30 minutes. For these solutions, absorbances A1, A2, A3, and A4 at a wavelength of 660 nm were measured using, as a control, a solution obtained by weighing 2 mL of a 0.2 mol/L hydrochloric acid reagent and performing the same operation as described above. The absorbances A1, A2, A3, and A4 were plotted on the vertical axis and the amount ($\mu$g) of tyrosine in 2 mL of each solution was plotted on the horizontal axis to prepare a calibration curve, and the amount ($\mu$g) of tyrosine with respect to the absorbance difference of 1 was determined.

The amount of enzyme that causes an increase in colored materials by Folin's reagent corresponding to 1 $\mu$g of tyrosine per minute was defined as 1 unit (1 U).

[Mathematical Formula 2]

$$\text{Protease activity (U/g, U/mL)} = (AT - AB) \times F \times 11/2 \times 1/10 \times 1/M$$

AT: Absorbance of enzymatic reaction solution
AB: Absorbance of blank
F: Tyrosine amount ($\mu$g) when absorbance difference determined from tyrosine calibration curve is 1
11/2: Conversion coefficient to total liquid amount after stop of reaction
1/10: Conversion coefficient per minute of reaction time
M: Amount (g or mL) of sample in 1 mL of sample solution

[Test Example 1]

A protease (component (B)), sorbitol (component (C)), or citric acid (component (D)) was added to and mixed with a protein glutaminase (PG, component (A)) purified by ion exchange chromatography in the amounts shown in Tables 1 to 3. The pH was adjusted to 6.0 (25°C) using 80% acetic acid or a 6 N aqueous sodium hydroxide solution as a pH adjuster, and liquid enzyme preparations having a PG concentration as shown in Tables 1 to 3 were prepared. The prepared liquid enzyme preparation was placed in a container, and nitrogen was injected from a nitrogen cylinder into the gas phase of the storage container (space above the liquid phase composed of the liquid enzyme preparation), and taking in and out was repeated 20 times. Oxygen in the container was almost completely replaced with nitrogen (that is, the oxygen in the gas phase is substituted with nitrogen without bubbling nitrogen into the liquid enzyme preparation), and the container was hermetically sealed to obtain a liquid enzyme preparation packed in a container.

The liquid enzyme preparation packed in the container thus obtained was left to stand at 40°C for 1 month. When the protein deamidase activity value before standing was regarded as 100%, a relative value (%) of the protein deamidase activity value after standing was calculated as residual activity. The results are shown in Tables 1 to 3. Fig. 1 shows a correlation between the blended protease addition activity and the residual activity of the protein deamidase activity.

[Table 1]

| | Reference Example 1 | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| (A) PG (U/mL) | | | | | | 500 | | | | | | |
| (B) Protease 1 (U/mL) | - | - | - | - | - | - | - | - | - | - | - | - |
| (B) Protease 2 (U/mL) | - | 0.6 | 6 | 60 | - | - | 0.6 | 6 | 60 | - | - | - |
| (B) Protease 3 (U/mL) | - | - | - | - | 0.6 | 6 | - | - | - | 0.6 | 6 | 60 |
| (C) Sorbitol (w/v%) | - | - | - | - | - | - | 40 | 40 | 40 | 40 | 40 | 40 |
| (D) 3Na citrate (w/v%) | - | - | - | - | - | - | 3 | 3 | 3 | 3 | 3 | 3 |
| pH adjuster | | | | | | q.s. | | | | | | |
| Water | | | | | | Remainder | | | | | | |
| Total (w/v%) | | | | | | 100 | | | | | | |
| pH (25°C) | | | | | | pH6.0 | | | | | | |
| (B) (U) per 100 U of (A) | | 0.12 | 1.2 | 12 | 0.12 | 1.2 | 0.12 | 1.2 | 12 | 0.12 | 1.2 | 12 |
| Residual activity | 100% | 100% | 100% | 89% | 100% | 100% | 81% | 81% | 78% | 86% | 89% | 80% |

Protease 1: derived from *Chryseobacterium proteolyticum*
Protease 2: derived from *Bacillus licheniformis*
Protease 3: derived from *Bacillus amyloliquefaciens*

[Table 2]

| | Example 12 | Example 13 | Example 14 | Example 15 | Example 16 | Example 17 | Example 18 | Example 19 | Example 20 | Comparative Example 2 | Comparative Example 3 |
|---|---|---|---|---|---|---|---|---|---|---|---|
| (A) PG (U/mL) | 500 | | | | | | | | | | |
| (B) Protease 1 (U/mL) | 1 | 3 | 6 | 7 | 17 | 19 | 82 | 123 | 265 | 4111 | 5642 |
| (B) Protease 2 (U/mL) | - | - | - | - | - | - | - | - | - | - | - |
| (B) Protease 3 (U/mL) | - | - | - | - | - | - | - | - | - | - | - |
| (C) Sorbitol (w/v%) | - | - | - | - | - | - | - | - | - | - | - |
| (D) 3Na citrate (w/v%) | - | - | - | - | - | - | - | - | - | - | - |
| pH adjuster | q.s. | | | | | | | | | | |
| Water | Remainder | | | | | | | | | | |
| Total (w/v%) | 100 | | | | | | | | | | |
| pH (25°C) | pH6.0 | | | | | | | | | | |
| (B) (U) per 100 U of (A) | 0.2 | 0.6 | 1.2 | 1.4 | 3.4 | 3.8 | 16.4 | 24.6 | 53 | 822.2 | 1128 |
| Residual activity | 100% | 100% | 100% | 100% | 100% | 100% | 100% | 86% | 82% | 2% | 1% |

Protease 1: derived from *Chryseobacterium proteolyticum*
Protease 2: derived from *Bacillus licheniformis*
Protease 3: derived from *Bacillus amyloliquefaciens*

EP 4 729 611 A1

[Table 3]

| | Example 21 | Example 22 | Example 23 | Comparative Example 4 | Comparative Example 5 | Example 24 | Example 25 | Example 26 | Example 27 | Comparative Example 6 |
|---|---|---|---|---|---|---|---|---|---|---|
| (A) PG (U/mL) | 500 | | | | | | | | | |
| (B) Protease 1 (U/mL) | 136 | 447 | 1432 | 2234 | 5962 | 85 | 282 | 331 | 1632 | 4767 |
| (B) Protease 2 (U/mL) | - | - | - | - | - | - | - | - | - | - |
| (B) Protease 3 (U/mL) | - | - | - | - | - | - | - | - | - | - |
| (C) Sorbitol (w/v%) | 40 | 40 | 40 | 40 | 40 | 40 | 40 | 40 | 40 | 40 |
| (D) 3Na citrate (w/v%) | - | - | - | - | - | 3 | 3 | 3 | 3 | 3 |
| pH adjuster | q.s. | | | | | | | | | |
| Water | Remainder | | | | | | | | | |
| Total (w/v%) | 100 | | | | | | | | | |
| pH (25°C) | pH6.0 | | | | | | | | | |
| (B) (U) per 100 U of (A) | 27.2 | 89.4 | 286.4 | 446.8 | 1192 | 17 | 56.4 | 66.2 | 326.4 | 953.4 |
| Residual activity | 100% | 95% | 76% | 32% | 8% | 90% | 90% | 82% | 68% | 8% |

Protease 1: derived from *Chryseobacterium proteolyticum*
Protease 2: derived from *Bacillus licheniformis*
Protease 3: derived from *Bacillus amyloliquefaciens*

As shown in Tables 1 to 3, from the comparison between Examples 1 to 27 and Comparative Examples 2 to 6, when the blending amount of the component (B) was about 2,000 U/mL or less (the blending amount of the component (B) was about 400 U or less with respect to 100 U of the component (A)), the activity stabilizing effect of the protein deamidase could be enhanced.

From the comparison between Example 1 and Example 6, the comparison between Example 2 and Example 7, the comparison between Example 3 and Example 8, the comparison between Example 4 and Example 9, the comparison between Example 5 and Example 10, and the comparison between Example 18 and Example 24, when the blending amount of the component (B) was relatively small, the residual activity tended to decrease by additionally blending the component (C), but from the comparison between Example 19 and Example 21 and the comparison between Example 20 and Example 25, when the blending amount of the component (B) was relatively large (specifically, when the blending amount of the component (B) was about 100 U/mL or more (the blending amount of the component (B) was about 20 U or more with respect to 100 U of the component (A))), the residual activity was improved by additionally blending the component (C). Note that, from the comparison between Example 21 and Example 24 and the comparison between Example 22 and Example 26, the residual activity tended to slightly decrease when the component (D) was blended, but when the blending amount of the component (B) was relatively large, the stability of the liquid enzyme preparation additionally blended with the component (C) was high, so that it was possible to suppress the decrease in the residual activity even when the component (D) was blended, and effectively high stability was obtained.

Note that the activity stabilizing effect of the protein deamidase shown in Tables 1 to 3 was improved as compared with a case where the oxygen in the container was not substituted with nitrogen, which was also excellent. Since such an excellent stabilizing effect was obtained even when the dissolved oxygen amount in the liquid enzyme preparation shown in Tables 1 to 3 was more than 3 mg/L (specifically, more than 3 mg/L and 8 mg/L or less), which shows that the stability of useful proteins is deteriorated in PTL 3, it was completely unexpected that such a stabilizing effect was obtained only by replacing the headspace with an inert gas regardless of the dissolved oxygen amount in the liquid enzyme preparation.

## Claims

1. A liquid enzyme preparation comprising: (A) a protein deamidase; and (B) a protease.

2. The liquid enzyme preparation according to claim 1, wherein the liquid enzyme preparation is accommodated in a container whose headspace is replaced with an inert gas.

3. The liquid enzyme preparation according to claim 1, further comprising (C) sorbitol.

4. The liquid enzyme preparation according to claim 1, further comprising (D) a citrate.

5. The liquid enzyme preparation according to claim 3, wherein a content of the component (C) is 30 w/v% or more.

6. The liquid enzyme preparation according to claim 4, wherein a content of the component (D) is 1 w/v% or more.

7. The liquid enzyme preparation according to claim 1, wherein a pH is 5.5 or more.

8. The liquid enzyme preparation according to claim 1, wherein a pH is 8.2 or less.

9. The liquid enzyme preparation according to claim 1, wherein a content of the component (A) is 0.1 to 10,000 U/ml.

10. The liquid enzyme preparation according to claim 1, wherein the component (A) is a protein deamidase derived from Chryseobacterium proteolyticum.

11. The liquid enzyme preparation according to claim 1, wherein a content of the component (B) is 0.01 to 2,000 U/mL.

12. The liquid enzyme preparation according to claim 1, wherein the component (B) is a bacteria-derived protease.

13. The liquid enzyme preparation according to claim 12, wherein the bacteria-derived protease is a protease derived from the genus Chryseobacterium.

14. The liquid enzyme preparation according to claim 12, wherein the bacteria-derived protease is a protease derived from the genus Bacillus and/or Geobacillus.

15. The liquid enzyme preparation according to claim 1, wherein a content of the component (B) per 100 U of the component (A) is 0.002 to 400 U.

FIG. 1

Correlation between protease addition activity and residual activity

## INTERNATIONAL SEARCH REPORT

| International application No. |
| --- |
| **PCT/JP2024/021776** |

| | |
| --- | --- |
| **A.** | **CLASSIFICATION OF SUBJECT MATTER** |

*C12N 9/80*(2006.01)i; *C12N 9/52*(2006.01)i; *C12N 9/54*(2006.01)i
FI:  C12N9/80; C12N9/54; C12N9/52

According to International Patent Classification (IPC) or to both national classification and IPC

| | |
| --- | --- |
| **B.** | **FIELDS SEARCHED** |

Minimum documentation searched (classification system followed by classification symbols)

C12N9/80; C12N9/52; C12N9/54

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| Y | WO 2022/118914 A1 (AMANO ENZYME INC.) 09 June 2022 (2022-06-09) claims 1, 3, 5-6, paragraphs [0006], [0029], [0030], [0035] | 1-15 |
| Y | WO 2022/215688 A1 (AMANO ENZYME EUROPE LTD.) 13 October 2022 (2022-10-13) paragraphs [0024], [0025] | 1-15 |
| Y | WO 2022/102723 A1 (AMANO ENZYME INC.) 19 May 2022 (2022-05-19) paragraphs [0008], [0037], [0041] | 1-15 |
| Y | JP 2021-145603 A (AJINOMOTO CO., INC.) 27 September 2021 (2021-09-27) claims 1, paragraphs [0006], [0015], [0040]) | 2, 4, 6 |
| A | | 1, 3, 5, 7-15 |
| Y | WO 2015/158723 A1 (NOVOZYMES A/S) 22 October 2015 (2015-10-22) examples | 13 |
| A | | 1-12, 14-15 |
| A | JP 2014-532421 A (VALIO LTD.) 08 December 2014 (2014-12-08) | 1-15 |

| ✓ | Further documents are listed in the continuation of Box C. | ✓ | See patent family annex. |
| --- | --- | --- | --- |

| | |
| --- | --- |
| \*     Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **22 August 2024** | **03 September 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2024/021776** |

| C. | DOCUMENTS CONSIDERED TO BE RELEVANT | |
| --- | --- | --- |
| Category* | Citation of document, with indication, where appropriate, of the relevant passages | Relevant to claim No. |
| A | JP 4-207194 A (AJINOMOTO CO., INC.) 29 July 1992 (1992-07-29) | 1-15 |

Form PCT/ISA/210 (second sheet) (July 2022)

**INTERNATIONAL SEARCH REPORT**
Information on patent family members

International application No.

**PCT/JP2024/021776**

| Patent document cited in search report | | | Publication date (day/month/year) | Patent family member(s) | | | Publication date (day/month/year) |
|---|---|---|---|---|---|---|---|
| WO | 2022/118914 | A1 | 09 June 2022 | US | 2024/0026332 | A1 | |
| | | | | claims 1, 3, 5-6, paragraphs [0007], [0030], [0031], [0036] | | | |
| | | | | EP | 4257682 | A1 | |
| | | | | CN | 116547381 | A | |
| WO | 2022/215688 | A1 | 13 October 2022 | US | 2024/0108029 | A1 | |
| | | | | paragraphs [0030], [0031] | | | |
| | | | | EP | 4321032 | A1 | |
| | | | | CN | 116916759 | A | |
| WO | 2022/102723 | A1 | 19 May 2022 | US | 2024/0016187 | A1 | |
| | | | | paragraphs [0008], [0046], [0050] | | | |
| | | | | EP | 4245149 | A1 | |
| | | | | CN | 116471944 | A | |
| JP | 2021-145603 | A | 27 September 2021 | (Family: none) | | | |
| WO | 2015/158723 | A1 | 22 October 2015 | US | 2017/0114301 | A1 | |
| | | | | EP | 3132032 | A1 | |
| | | | | CN | 106164264 | A | |
| JP | 2014-532421 | A | 08 December 2014 | US | 2014/0287098 | A1 | |
| | | | | WO | 2013/064736 | A1 | |
| | | | | EP | 2773753 | A1 | |
| | | | | CN | 104024406 | A | |
| | | | | KR | 10-2014-0096090 | A | |
| JP | 4-207194 | A | 29 July 1992 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010029685 A
- WO 2013064736 A
- JP 2013049650 A
- JP 2000050887 A
- JP 2001218590 A
- WO 2006075772 A1
- WO 2015133590 A1